# EUROPEAN PATENT APPLICATION

(11) **EP 1 229 131 A1**
(43) Date of publication of application: **07.08.2002**
(21) Application number: 01956887.2
(22) Date of filing: 14.08.2001
(51) Int. Cl.: C12Q 1/68

(54) **METHOD OF DETECTING GENE**

(30) Priority: 30.08.2000 JP 2000261687
(71) Applicant: SANKO JUNYAKU CO., LTD., Tokyo 101-0032 (JP)
(72) Inventor: USUI, Mitsugu, Abiko-shi, Chiba 270-1176 (JP); MITSUKA, Mari, Kashiwa-shi, Chiba 277-0843 (JP); HAKII, Chikako, Kashiwa-shi, Chiba 277-0841 (JP)
(74) Representative: Grünecker, August, Dipl.-Ing.
(86) International application number: JP0107020
(87) International publication number: WO0218642

(57) **Abstract**

The present invention provides a gene detecting method making it possible to detect a gene in response to formation of a polymer not requiring an operation for catching linked oligonucleotides nor an operation for washing to remove surplus oligonucleotides by making use of the PALSAR method. This method comprises the steps of providing a plurality of pairs of probes, each probe comprising n (n ≧ 3) base sequence regions complementary to each other; previously cleaving at least one region and/or a partner or partners thereof at least at one site, the region or the regions being complementary to a target gene of the pair of probes; performing hybridization reactions, ligation reactions and separation reactions under thermal control; ligating the cleaved probes to form complete probes; and forming a double-stranded polymer by means of a probe-polymer forming method to detect the target gene.

## Description

### Technical Field:

The present invention relates to a method of detecting a gene by making use of a method for forming a probe-polymer in which a double-stranded polymer is formed by providing a plurality of pairs of probes, each probe comprising n (n ≧ 3) base sequence regions complementary to each other (may be referred to a "HoneyComb probe: HCP" hereinafter), and hybridizing the probes so as to cross alternately (see USP No. 6,261,846, Japanese Patent Laid-Open Publication No. 2000-201687, and Japanese Patent Application No. 2000-98797, and this method may be referred to a "PALSAR (Probe Alternation Link Self-Assembly Reaction) method" hereinafter).

### Background Art:

There has been developed a method of detecting a target gene or a single nucleotide polymorphism by hybridizing two oligonucleotides complementary to the target gene or the single nucleotide polymorphism to the target gene or the single nucleotide polymorphism so that the two oligonucleotides adjoin to each other and causing a ligation reaction for linking the two oligonucleotides to each other.

The ligation reaction described above is largely classified to one method in which ligase is used and another method in which a specific oligonucleotide is used in place of an enzyme.

In the method in which the ligase is used, there are employed T4 DNA ligase used in the general gene manipulation, and thermostable DNA ligase (Thermus thermophilus DNA ligase) used in a LCR (Ligase Chain Reaction) method (see USP No. 5,792,607 and USP No. 5,494,810). In an autoligation reaction method in which any enzyme is not used (see Yanzheng Xu and Eric T. Kool. (1999) Nucleic Acids Res., 27. 875-881), two oligonucleotides are linked to each other by previously bonding phosphorothioate and iodothymidine to adjoining 3' end and 5' end each of two oligonucleotides to achieve 5'-bridging phosphorothioate linkage.

There are various methods for linking two oligonucleotides to each other as described above, but there are few methods of detecting linked oligonucleotides, and only a way to detect a target gene is performed, as represented by the LCR™, by catching only the linked oligonucleotides with a specific device and washing to remove surplus oligonucleotides by means of B/F separation.

As a method of enabling a fluorescent substance, fluorescence of which has been suppressed by a quencher, to separate from the quencher and start emitting fluorescence, a TaqMan PCR method or an invader method is available. Separation of the fluorescent substance from the quencher and start of emission of fluorescence are enabled by cleaving a probe to which the fluorescent substance is linked by making use of the 5' endonuclease activity of Taq DNA polymerase in the TaqMan PCR method and by cleaving a probe to which the fluorescent substance is linked with an enzyme (cleavase) having specific endonuclease activity to recognize DNA structure and break it in the invader method, and thus a specific enzyme is required in both methods.

On the other hand, the present inventors have invented and proposed an epoch-making method enabling efficient polymerization of probes without using any enzyme and branched DNA, namely the PALSAR method (see USP No. 6,261,846, Japanese Patent Laid-Open Publication No. 2000-201687, and Japanese Patent Application No. 2000-98797).

### Disclosure of the Invention:

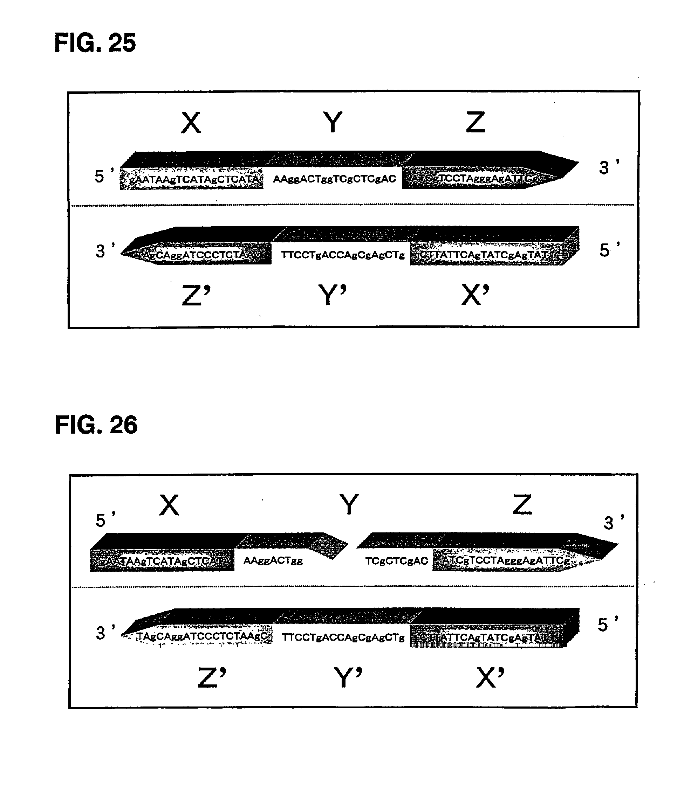

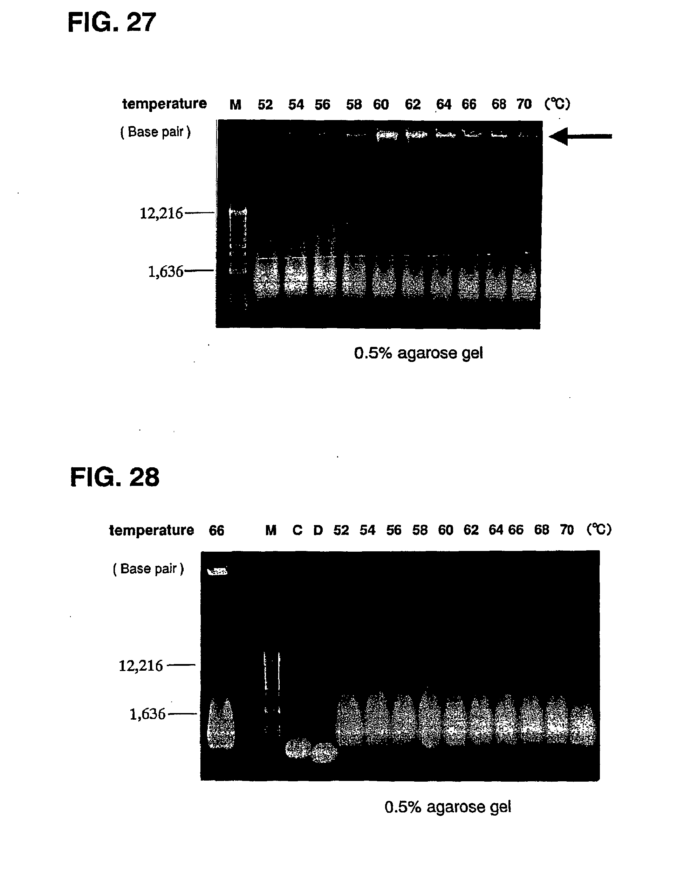

In the light of the current situation of the conventional art as described above, the present inventors made strenuous efforts to invent a new method of detecting a gene by making use of the PALSAR method described above, and found out the fact as described in Experimental Example 1 below that, when a region complementary to a target gene of a pair of probes used in the PALSAR method has not been cleaved as shown in Fig. 25, a polymer is formed depending on temperature of the hybridization reaction as shown by the arrow in Fig. 27, but that, when the region complementary to the target gene of the pair of probes used in the PALSAR method has been cleaved as shown in Fig. 26, the polymer is not formed irrespective of any temperature of the hybridization reaction as shown in Fig. 28.

The present inventors found out that, by making use of the characteristics of the pair of probes used in the PALSAR method as described above, probes capable of forming the polymer only when a target gene is present, without the need of separating oligonucleotides having been bound to the target gene from surplus ones not having been bound to the target gene, can be produced, and that the gene can easily be detected by using the probes. The invention described herein is based on this finding.

It is an object of the present invention to provide a method of detecting a gene; making it possible to detect the gene in response to formation of a polymer by making use of the PALSAR method without the need of performing operations for catching linked oligonucleotides and washing to remove surplus oligonucleotides; and also making it possible to detect the gene without the need of any specific enzyme in response to fluorescence quenching caused by a quencher only where the double-stranded polymer is formed according to the PALSAR method by labeling 5' end of a pair of probes used in the PALSAR method with fluorescent substance and attaching 3' end thereof to the quencher.

To solve the problems as described above, according to a first aspect of the gene detecting method of the present invention, there is provided a gene detecting method by making use of a probe-polymer forming method for forming a double-stranded polymer by providing a plurality of pairs of probes, each probe comprising n (n ≧ 3) base sequence regions complementary to each other, and hybridizing the probes so as to cross alternately, wherein the gene detecting method comprises the steps of: providing the plurality of pairs of probes, wherein one probe of the pair of probes comprises at least one complementary region having base sequence complementary to a part of a target gene; previously cleaving the region or the regions and/or a partner or partners thereof at least at one site, the region or the regions being complementary to the target gene of the pair of probes; performing hybridization reactions, ligation reactions and separation reactions under thermal control; ligating the cleaved probes to form complete probes; and forming the double-stranded polymer by means of the probe-polymer forming method to detect the target gene.

More specifically, in the gene detecting method according to the present invention, the probes having a region complementary to the target gene with the region having been cleaved hybridize, when the target gene is present, to the target gene, and are ligated by a ligation reaction, and then are heated to separate the two strands. Then the probes still in the cleaved state hybridize to the target gene or the ligated probes, and are ligated by the ligation reaction, and then are heated to separate the two strands. These three steps, the hybridization reaction → the ligation reaction → the separation reaction, can be carried out repetitively by controlling the temperature to join the probes, thus complete probes being formed. The ligated probes form the double-stranded polymer according to the PALSAR method. When the target gene is absent, the cleaved probes are not joined to each other, and the polymer is not formed with the cleaved probes. Therefore, the target gene can be detected in response to formation of the polymer.

Preferably the ligation reactions should be performed by means of DNA ligase, thermostable DNA ligase or autoligation not using any enzyme.

A single-stranded DNA or RNA can be used for the target gene.

A double-stranded DNA or RNA can be used for the target gene.

It is preferable to design the previously cleaved sites of the probes to be complementary to single nucleotide polymorphisms (described as SNPs hereinafter).

It is advantageous to use a device for controlling reaction temperatures in the method.

Further it is preferable to bind quenchers (substances for fluorescence quenching) and fluorescent substances (fluorophores) to one or both ends of the pair of probes used, and to detect formation of the double-stranded polymer in response to fluorescence quenching.

### Brief Description of the Drawings:

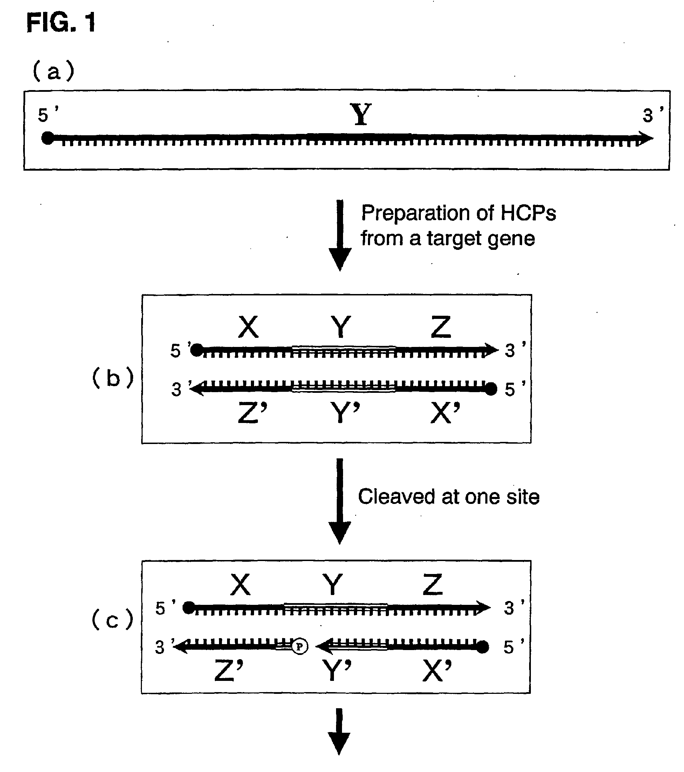

Fig. 1 is a schematic view showing an example of detection of a single-stranded target gene by making use of ligation reactions of a pair of probes used in the PALSAR method with one site thereof having been cleaved, and in this figure are shown the single-stranded target gene at (a), the pair of probes with one probe having a Y' region complementary to a Y region of the target gene at (b), and the pair of probes with the Y' region complementary to the target gene being cleaved at one site at (c), respectively.

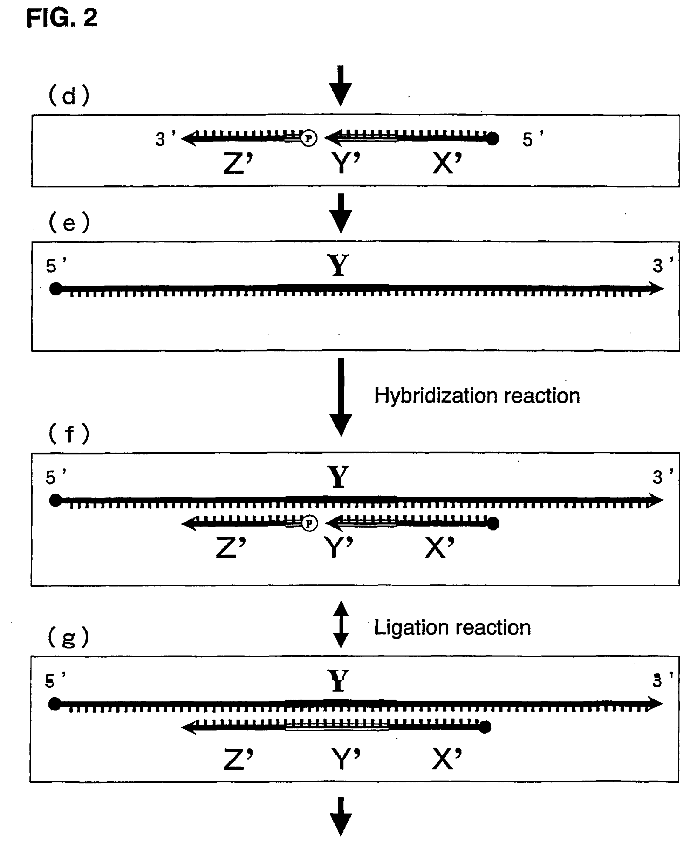

Fig. 2 is a schematic view showing the example of detection of the single-stranded target gene by making use of the ligation reactions of the pair of probes used in the PALSAR method with the one site thereof having been cleaved, and in this figure are shown the probe with the Y' region complementary to the target gene being cleaved at one site at (d), the single-stranded target gene at (e), and a hybridization reaction between the cleaved probe and the target gene at (f) and a ligation reaction of the cleaved probe at (g), respectively.

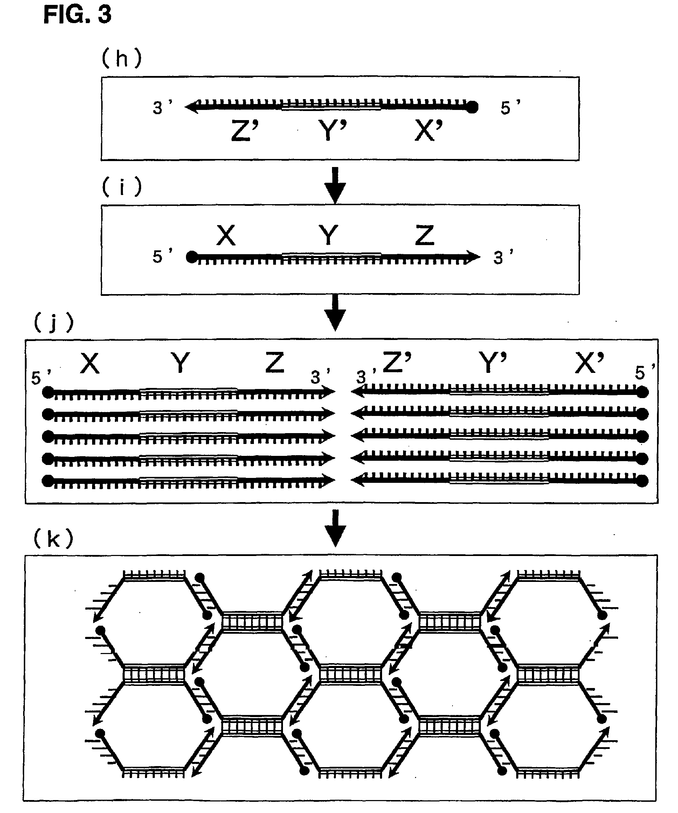

Fig. 3 is a schematic view showing the example of detection of the single-stranded target gene by making use of the ligation reactions of the pair of probes used in the PALSAR method with the one site thereof having been cleaved, and in this figure are shown the ligated probe at (h), another probe of the pair of HCPs at (i), a plurality of pairs of probes each comprising the ligated probes (j), and formation of a polymer based on the principle of the PALSAR method at (k), respectively.

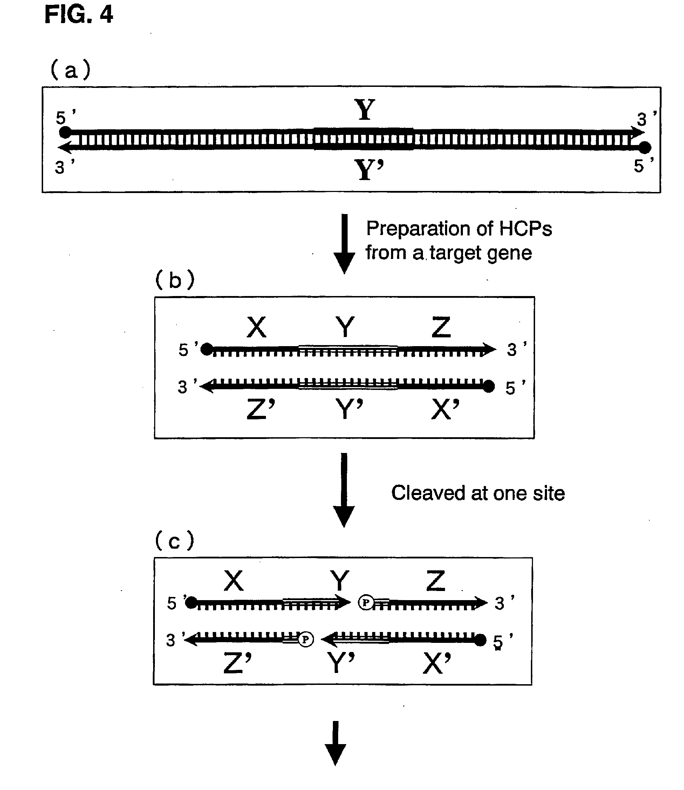

Fig. 4 is a schematic view showing an example of detection of a double-stranded target gene by making use of ligation reactions of a pair of probes used in the PALSAR method with one site thereof having been cleaved, and in this figure are shown the double-stranded target gene at (a), the pair of probes, each probe having regions complementary to a Y region or a Y' region of the double-stranded target gene at (b), and the pair of probes with each region complementary to the double-stranded target gene being cleaved at one site at (c), respectively.

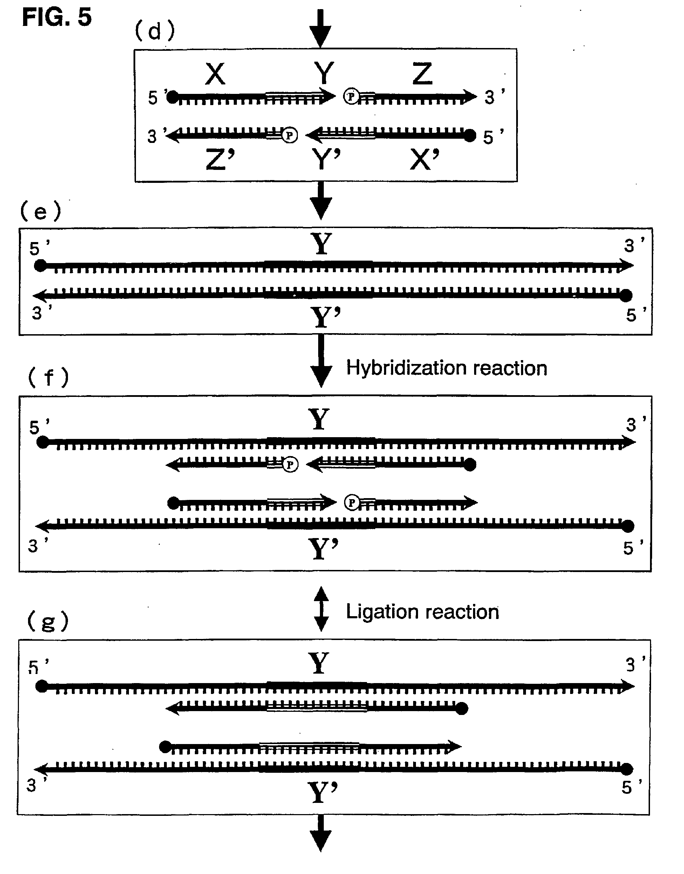

Fig. 5 is a schematic view showing the example of detection of the double-stranded target gene by making use of the ligation reactions of the pair of probes used in the PALSAR method with the one site thereof having been cleaved, and in this figure are shown the probes with the Y region and the Y' region complementary to the target gene being cleaved at one site each of the regions at (d), the target gene in which the two strand are separated at (e), a hybridization reaction between the cleaved probe and the separated target gene at (f), and a ligation reaction of the cleaved probes at (g), respectively.

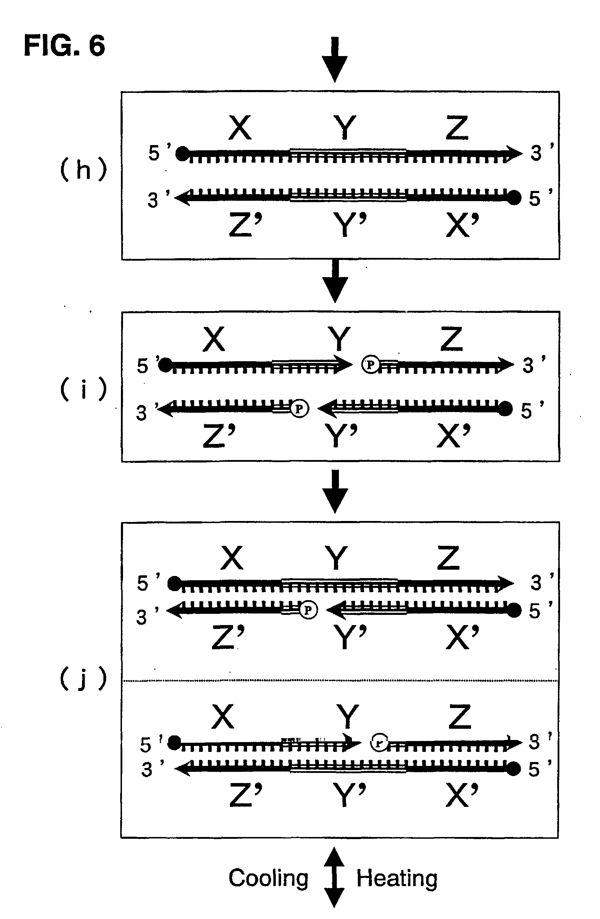

Fig. 6 is a schematic view showing the example of detection of the double-stranded target gene by making use of the ligation reactions of the pair of probes used in the PALSAR method with the one site thereof having been cleaved, and in this figure are shown the ligated probes at (h), the cleaved probes at (i), and a hybridization reaction between the ligated probes and the cleaved probes at (j), respectively.

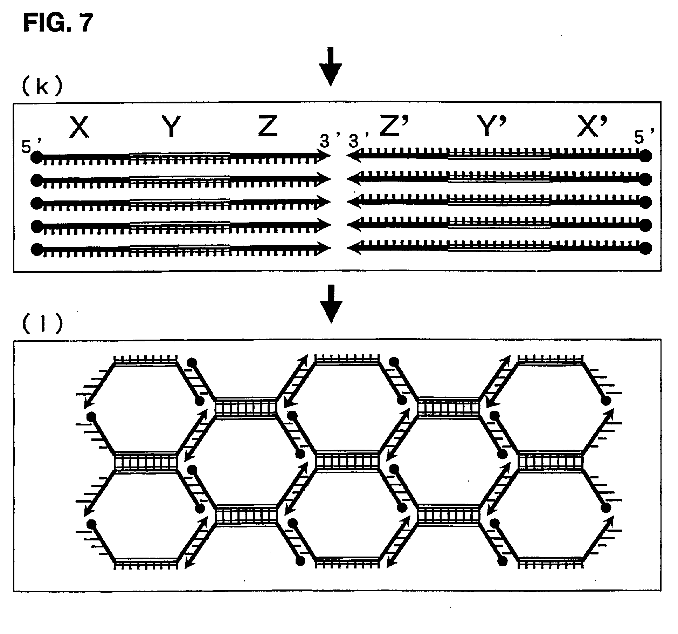

Fig. 7 is a schematic view showing the example of detection of the double-stranded target gene by making use of the ligation reactions of the pair of probes used in the PALSAR method with the one site thereof having been cleaved, and in this figure are shown a plurality of pairs of the ligated probes at (k), and formation of a polymer based on the principle of the PALSAR method (l), respectively.

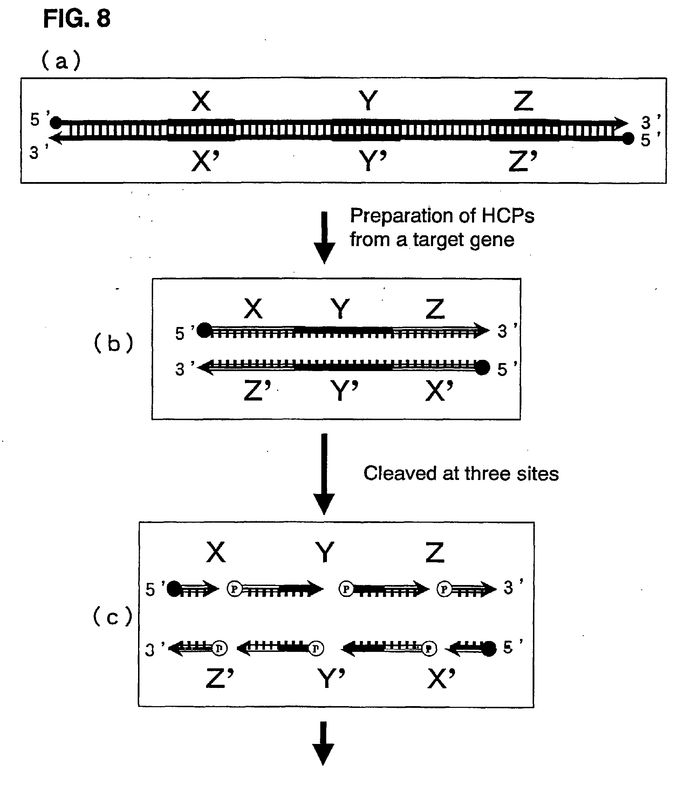

Fig. 8 is a schematic view showing an example of detection of a double-stranded target gene by making use of ligation reactions of a pair of probes used in the PALSAR method with three sites thereof having been cleaved, and in this figure are shown the double-stranded target gene at (a), the pair of probes, each probe having regions complementary to an X region or an X' region, a Y region or a Y' region, and a Z region or a Z' region of the double-stranded target gene at (b), and the pair of probes with the three regions complementary to the target gene being cleaved at one site each of the regions (at three sites in all) at (c), respectively.

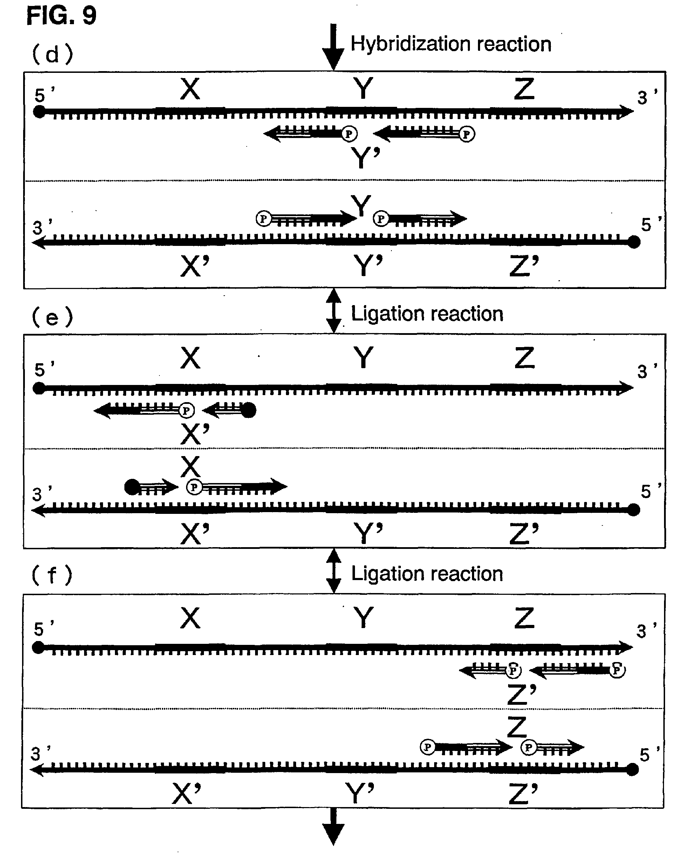

Fig. 9 is a schematic view showing the example of detection of the double-stranded target gene by making use of the ligation reactions of the pair of probes used in the PALSAR method with the three sites thereof having been cleaved, and in this figure are shown a hybridization reaction between the Y region of the target gene and the Y' region of the cleaved probe and also one between the Y' region of the target gene and the Y region of the cleaved probe at (d), a hybridization reaction between the X region of the target gene and the X' region of the cleaved probe and also one between the X' region of the target gene and the X region of the cleaved probe at (e), and a hybridization reaction between the Z region of the target gene and the Z' region of the cleaved probe and also one between the Z' region of the target gene and the Z region of the cleaved probe at (f), respectively.

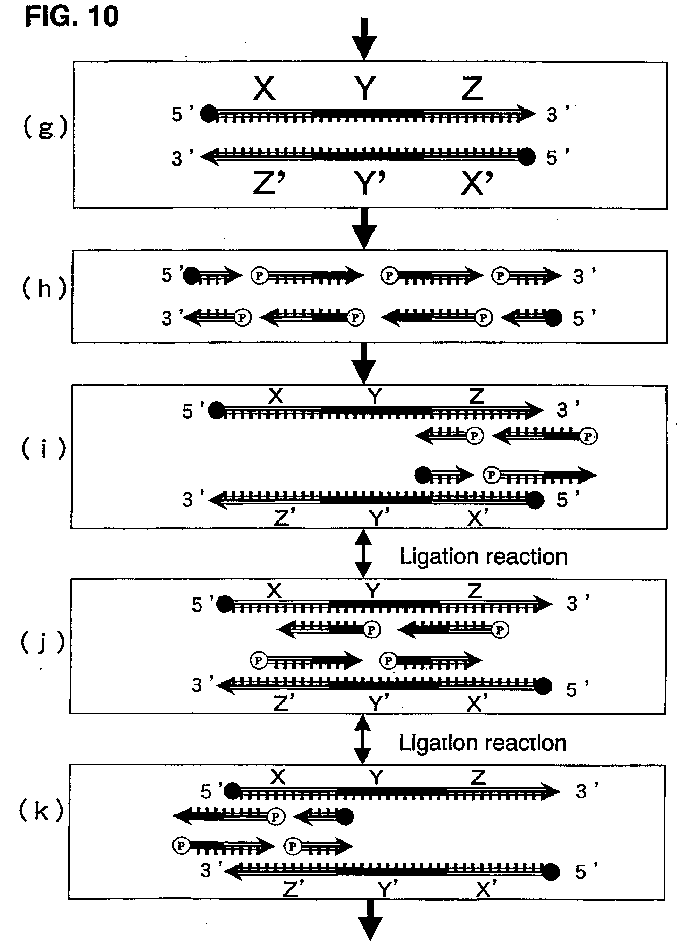

Fig. 10 is a schematic view showing the example of detection of the double-stranded target gene by making use of the ligation reactions of the pair of probes used in the PALSAR method with the three sites thereof having been cleaved, and in this figure are shown a pair of ligated probes at (g), the cleaved probes at (h), a hybridization reaction between the Z region of the ligated probe and the Z' region of the cleaved probe and also one between the X' region of the ligated probe and the X region of the cleaved probe at (i), a hybridization reaction between the Y region of the ligated probe and the Y' region of the cleaved probe and also one between the Y' region of the ligated probe and the Y region of the cleaved probe at (j), and a hybridization reaction between the X region of the ligated probe and the X' region of the cleaved probe and also one between the Z' region of the ligated probe and the Z region of the cleaved probe at (k), respectively.

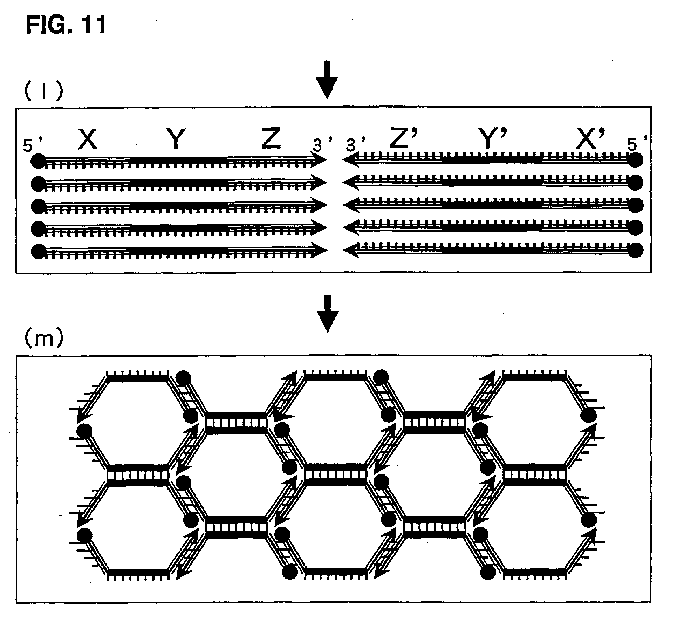

Fig. 11 is a schematic view showing the example of detection of the double-stranded target gene by making use of the ligation reactions of the pair of probes used in the PALSAR method with the three sites thereof having been cleaved, and in this figure are shown a plurality of pairs of the ligated probes at (l), and formation of a polymer based on the principle of the PALSAR method at (m), respectively.

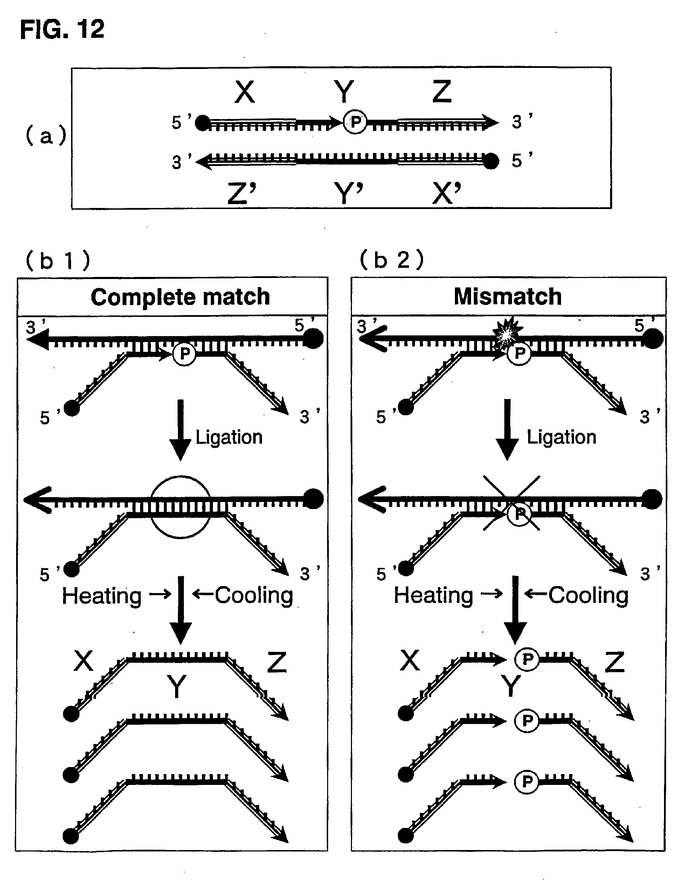

Fig. 12 is a schematic view showing an example of detection of SNPs by making use of ligation reactions of a pair of probes used in the PALSAR method with one site thereof having been cleaved, and in this figure are shown the pair of probes having a region (a Y region) complementary to a target gene containing SNPs with the region having been cleaved at one site at (a), hybridization reactions and ligation reactions in the case where SNPs of the target gene containing SNPs completely match (Complete match) a joined part of the probe with one site thereof having been cleaved upon hybridization at (b1), and hybridization reactions and ligation reactions in the case where SNPs of the target gene containing SNPs do not completely match (Mismatch) the joined part of the probe with one site thereof having been cleaved at (b2), respectively.

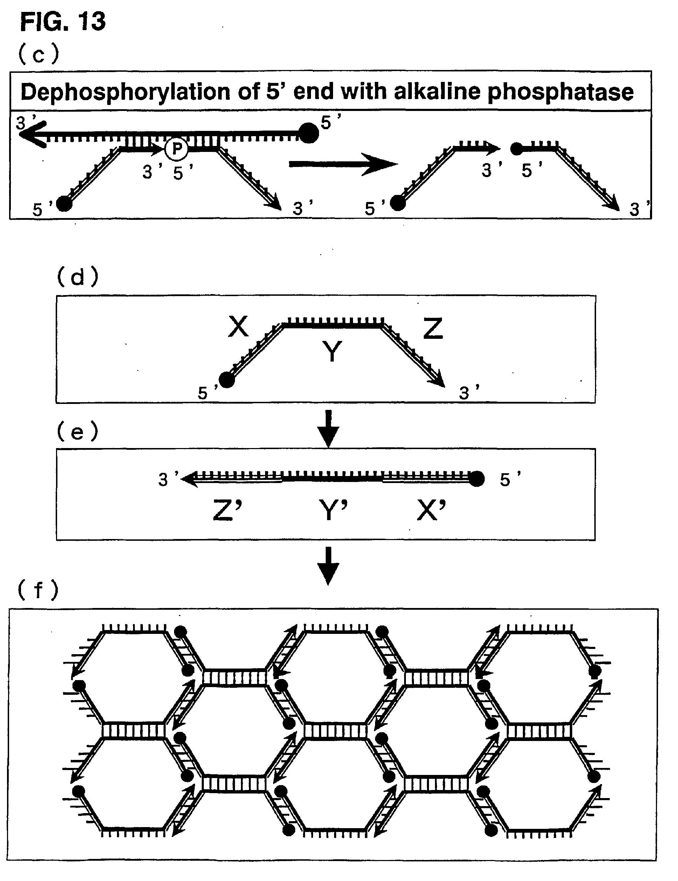

Fig. 13 is a schematic view showing the example of detection of SNPs by making use of the ligation reactions of the pair of probes used in the PALSAR method with the one site thereof having been cleaved, and in this figure are shown a dephosphorylation of probes not having been involved in the ligation reaction with alkaline phosphatase at (c), the ligated probes at (d), another probe not having been cleaved in the pair of HCPs at (e), and formation of a polymer based on the principle of PALSAR method at (f), respectively.

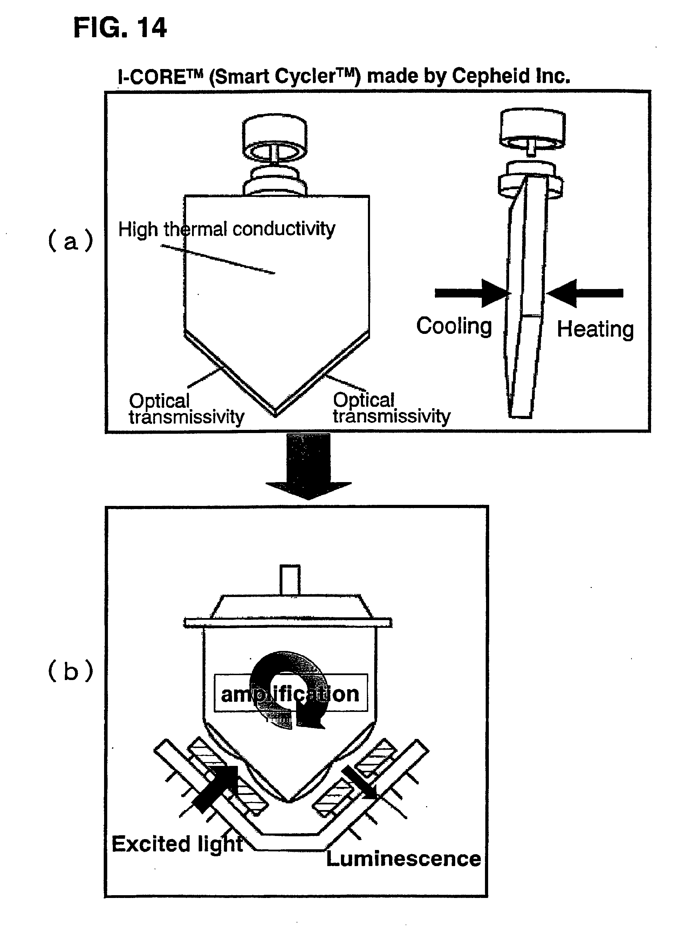

Fig. 14 is an explanatory view showing the principle of the I-CORE™ (Smart Cycler™) made by Cepheid Inc., and in this figure are shown a vessel having approximately pentagonal side faces and repetition of cooling and heating cycles via the sidewall at (a), and a method for checking the presence of fluorescent substance at (b), respectively.

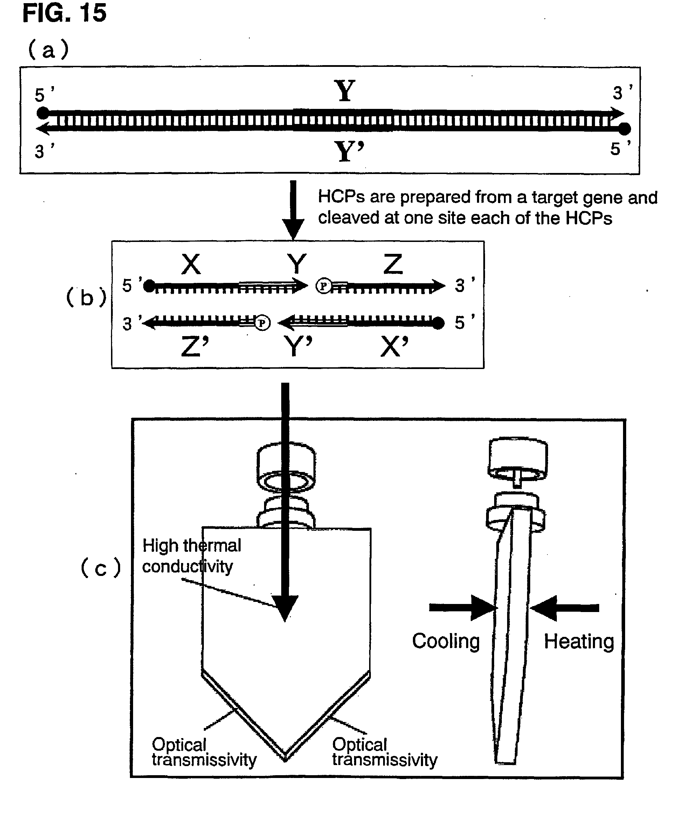

Fig. 15 is a schematic view showing an example of detection of a double-stranded target gene by making use of ligation reactions of a pair of probes used in the PALSAR method with one site thereof having been cleaved, which performed with the I-CORE™ (Smart Cycler™) made by Cepheid Inc., and in this figure are shown the double-stranded target gene at (a), the pair of probes, each probe having region complementary to a Y region or a Y' region of the double-stranded target gene with the complementary regions each having been cleaved at one site at (b), and a ligation reaction performed by using the I-CORE™ (Smart Cycler™) made by Cepheid Inc. at (c), respectively.

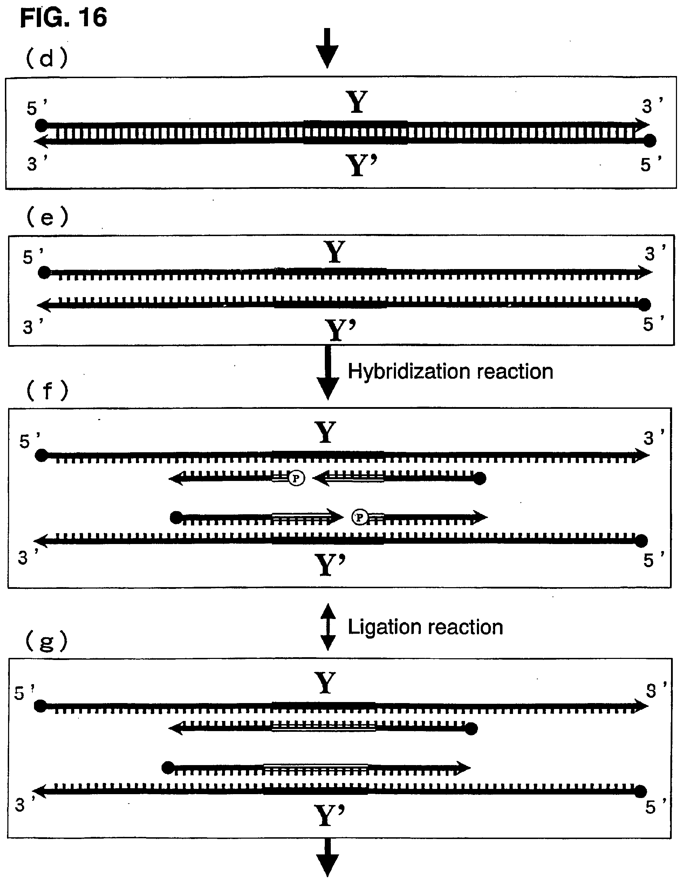

Fig. 16 is a schematic view showing the example of detection of the double-stranded target gene by making use of the ligation reactions of the pair of probes used in the PALSAR method with the one site thereof having been cleaved, which performed with the I-CORE™ (Smart Cycler™) made by Cepheid Inc., and in this figure are shown the double-stranded target gene at (d), the target gene in which the two strands have been separated at (e), a hybridization reaction between the target gene and the cleaved probes at (f), and a ligation reaction of the cleaved probes (g), respectively.

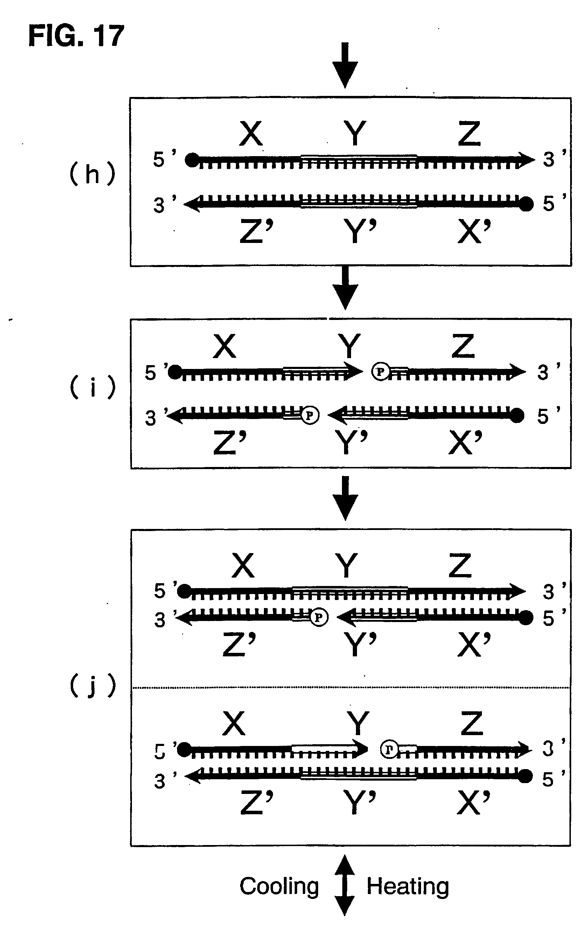

Fig. 17 is a schematic view showing the example of detection of the double-stranded target gene by making use of the ligation reactions of the pair of probes used in the PALSAR method with the one site thereof having been cleaved, which performed with the I-CORE™ (Smart Cycler™) made by Cepheid Inc., and in this figure are shown a pair of the ligated probes at (h), a pair of the cleaved probes at (i), and a hybridization reaction between the ligated probes and the cleaved probes at (j), respectively.

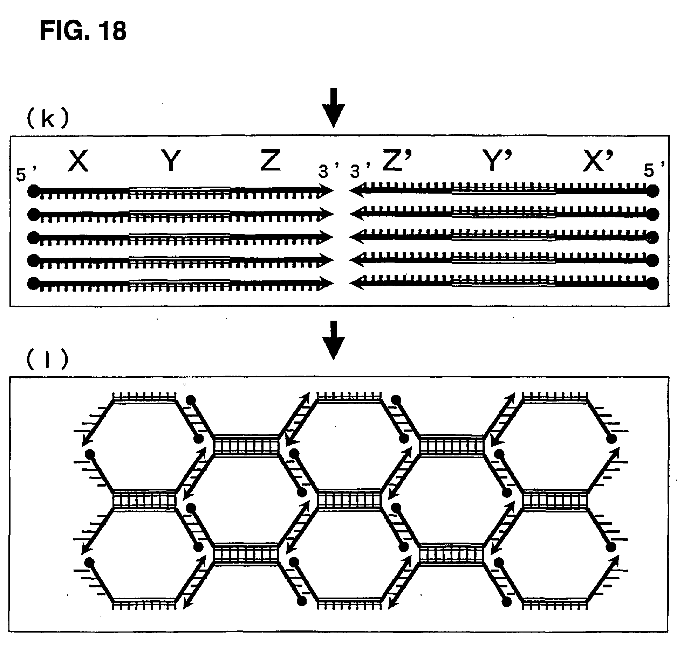

Fig. 18 is a schematic view showing the example of detection of the double-stranded target gene by making use of the ligation reactions of the pair of probes used in the PALSAR method with the one site thereof having been cleaved, which performed with the I-CORE™ (Smart Cycler™) made by Cepheid Inc., and in this figure are shown a plurality of pairs of the ligated probes at (k), and formation of a polymer based on the principle of the PALSAR method at (l), respectively.

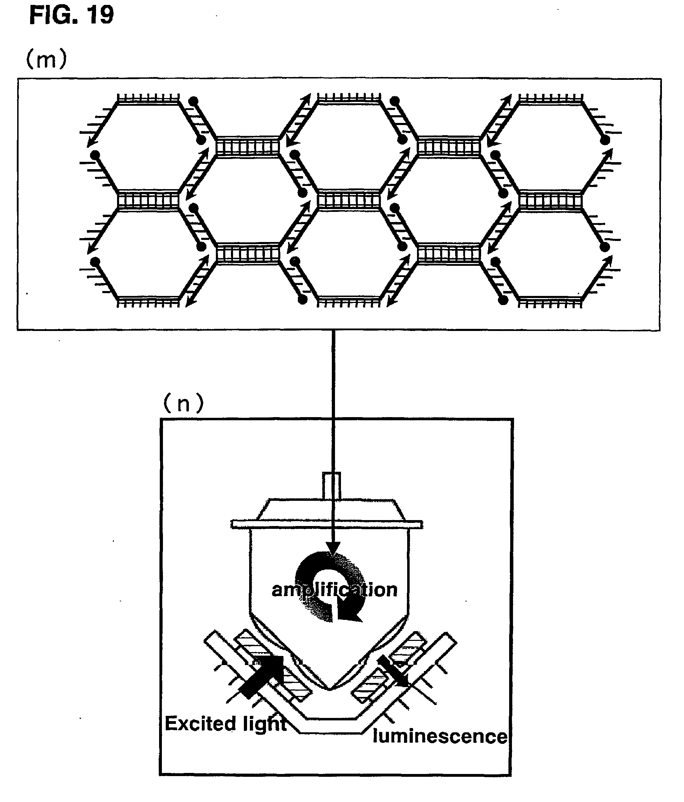

Fig. 19 is a schematic view showing the example of detection of the double-stranded target gene by making use of the ligation reactions of the pair of probes used in the PALSAR method with the one site thereof having been cleaved, which performed with the I-CORE™ (Smart Cycler™) made by Cepheid Inc., and in this figure are shown the polymer formed according to the principle of the PALSAR method at (m), and a method of detecting the polymer in the response to fluorescence by the use of the I-CORE™ (Smart Cycler™) made by Cepheid Inc. at (n), respectively.

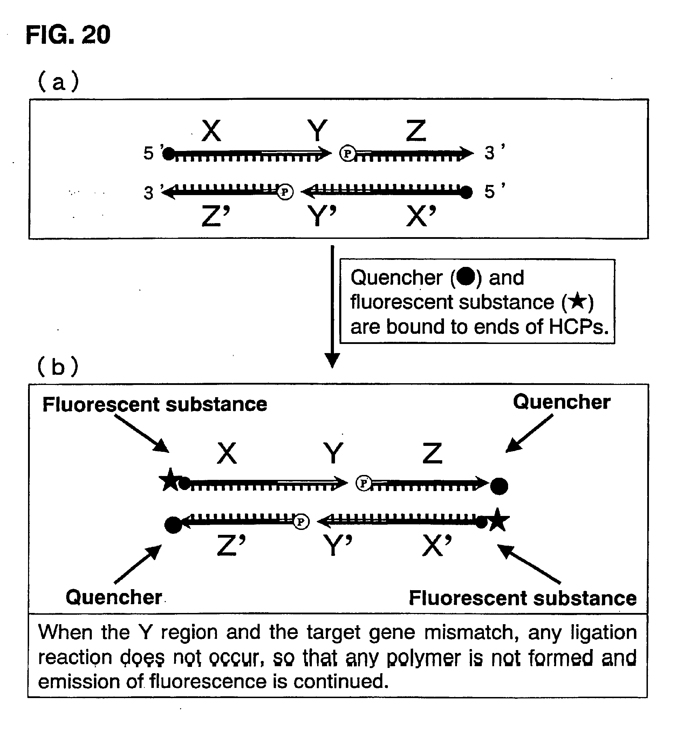

Fig. 20 is a schematic view showing an example of detection of a polymer formed according to the PALSAR method by the use of Molecular Beacons (in the case of Mismatch), and in this figure are shown a pair of HCPs, each probe used in the PALSAR method being cleaved at one site of a region (a Y region or a Y' region) complementary to a target gene at (a), and a pair of cleaved probes, 5' ends of the HCPs being labeled with fluorescent-substances and 3' ends thereof being bound to quenchers at (b), respectively.

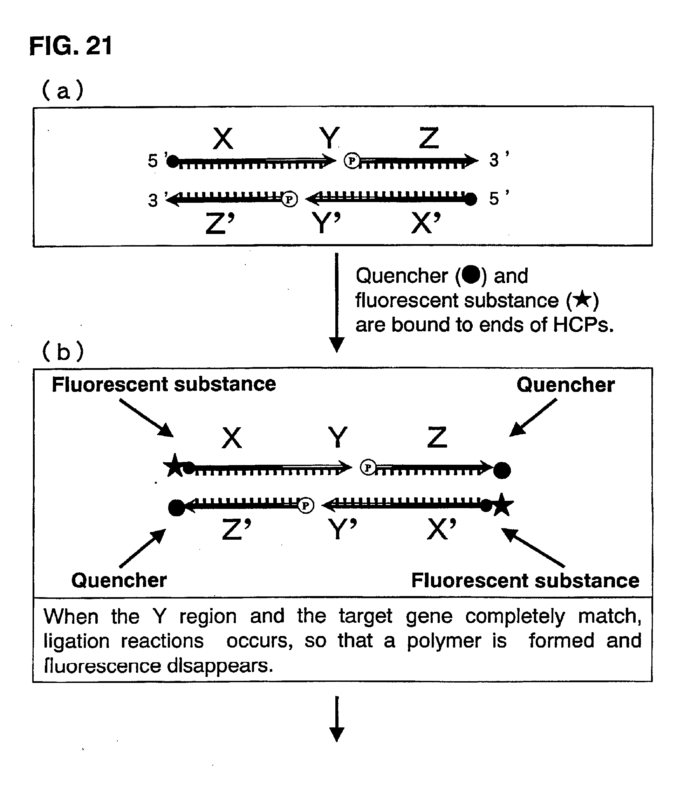

Fig. 21 is a schematic view showing an example of detection of a polymer formed according to the PALSAR method by the use of Molecular Beacons (in the case of Complete match), and in this figure are shown a pair of HCPs, each probe used in the PALSAR method being cleaved at one site of a region (a Y region or a Y' region) complementary to a target gene at (a), and a pair of cleaved probes, 5' ends of the HCPs being labeled with fluorescent-substances and 3' ends of the HCPs being bound to quenchers at (b), respectively.

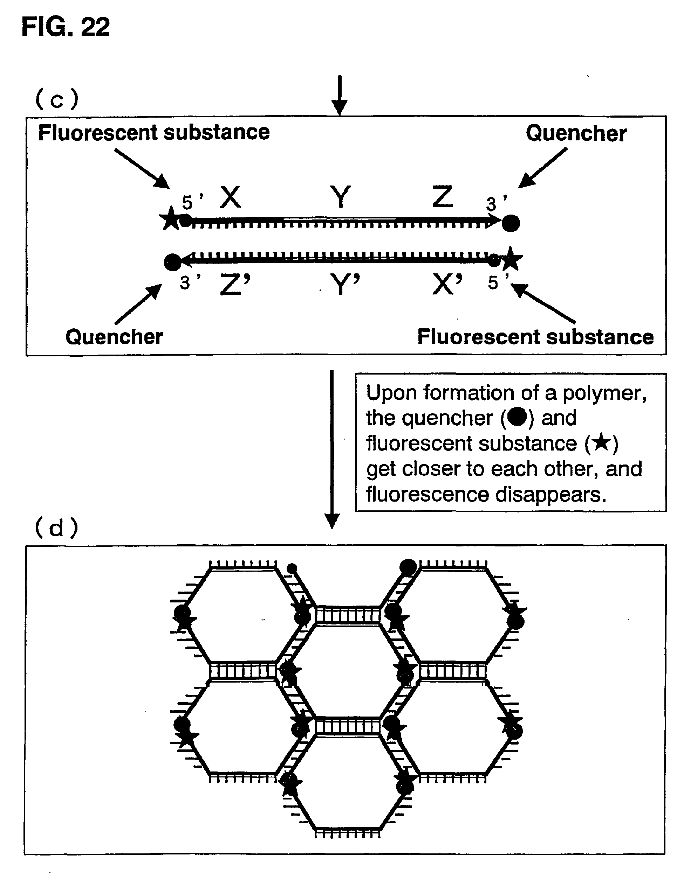

Fig. 22 is a schematic view showing the example of detection of the polymer formed according to the PALSAR method by the use of Molecular Beacons (in the case of Complete match), and in this figure are shown a pair of probes ligated by means of a ligation reaction at (c), and fluorescent quenching by a polymer formed according to the principle of the PALSAR method at (d), respectively.

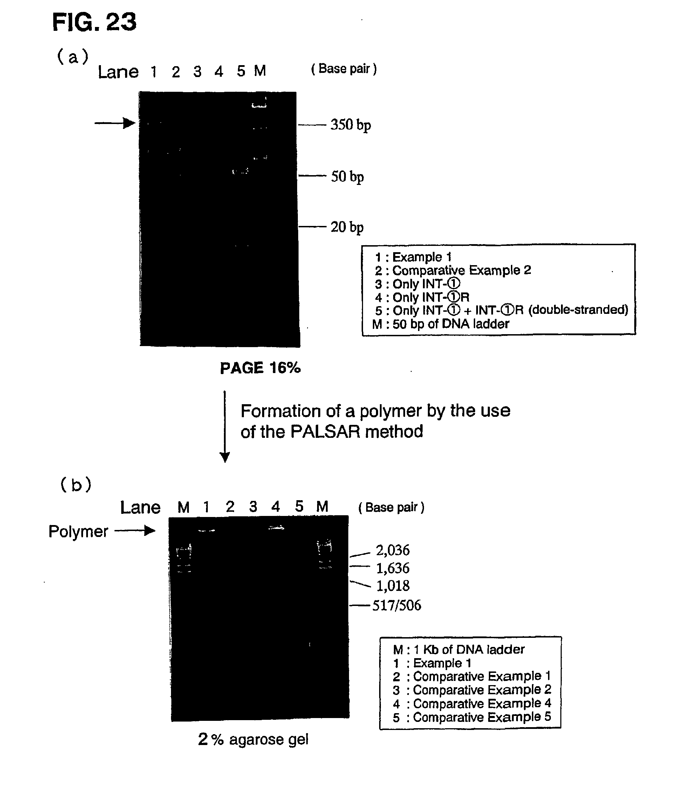

Fig. 23 shows photographs showing results of Example 1, Comparative Example 1, Comparative Example 2, Comparative Example 4, and Comparative Example 5 respectively, and in this figure are shown results by PAGE at (a), and results by agarose gel electrophoresis at (b), respectively.

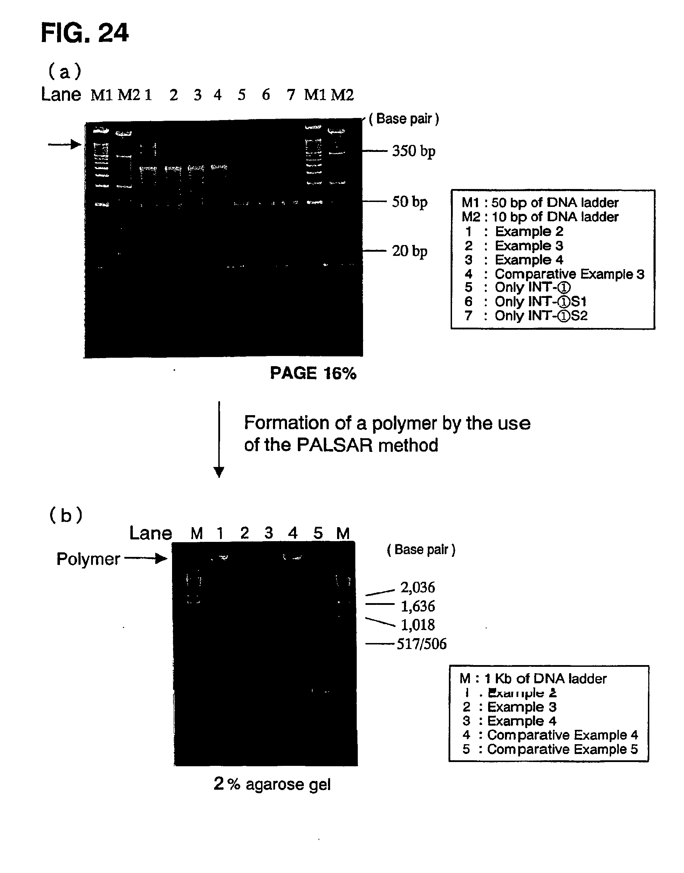

Fig. 24 shows photographs showing results of Examples 2 to 4 and Comparative Examples 3 to 5, and in this figure are shown results by PAGE at (a), and results by agarose gel electrophoresis at (b), respectively.
Fig. 25 is a schematic view showing DNA probes used in Experimental Example 1 in which a region complementary to a target gene of a pair of probes used in the PALSAR method has not been cleaved.
Fig. 26 is a schematic view showing DNA probes used in Experimental Example 1 in which the region complementary to the target gene of the pair of probes used in the PALSAR method has been cleaved.
Fig. 27 shows a photograph for agarose gel electrophoresis showing results of Experimental Example 1 in the case that the region complementary to the target gene of the pair of probes used in the PALSAR method has not been cleaved.
Fig. 28 shows a photograph for agarose gel electrophoresis showing results of Experimental Example 1 in the case that the region complementary to the target gene of the pair of probes used in the PALSAR method has been cleaved.

### Best Mode to Carry Out the Invention:

Embodiments of the present invention are described below with reference to the attached drawings, and it is needless to say that these embodiments are given for an illustrative purpose and that various modifications are possible within a scope of the technological idea according to the present invention.

Ligation reactions used in the embodiments described hereinafter are not limited to methods in which ligase is used, and nor to methods in which a specific oligonucleotide is used in place of an enzyme.

Fig. 1 to Fig. 3 are schematic views each showing an example of detection of a single-stranded target gene by making use of ligation reactions of a pair of probes used in the PALSAR method with one site thereof having been cleaved.

As shown at (a) and (b) in Fig. 1, from the single-stranded target gene, an HCP having a region (a Y' region) complementary to the target gene and an HPC as a partner for the former one are prepared (Fig. 1, (b)), and the Y' region of the probe having the region complementary to the Y region of the target gene (Fig. 1, (b)) is previously cleaved at one site as shown at (c) in Fig. 1. The cleaved probe (Fig. 2, (d)) hybridizes to the target gene as shown at (f) in Fig. 2, and then is ligated by means of a ligation reaction as shown at (g) in Fig. 2.

Then the ligated probe (Fig. 3, (h)) and the cleaved probe (Fig. 2, (d)) are reacted so as to carry out repetitively cycles of separation, hybridization, and ligation reactions under thermal control in the range of 94 °C to 25 °C (Fig. 2, (f), (g), and Fig. 3, (h)), and then the ligated probes (Fig. 3, (h)) react with the other HCPs in a pair additionally added thereto (Fig. 3, (i), (j)) to form a polymer based on the principle of the PALSAR method as shown at (k) in Fig. 3.

The formed polymer can easily be detected by the general agarose gel electrophoresis or other detecting method described below (Fig. 20 to Fig. 22).

It should be noted that a pair of probes being cleaved a region or regions and a partner or partners thereof, the region or the regions being complementary to the target gene, may be used.

Fig. 4 to Fig. 7 are schematic views each showing an example of detection of a double-stranded target gene by making use of ligation reactions of a pair of probes used in the PALSAR method with one site thereof having been cleaved.

As shown at (a) and (b) in Fig. 4, from the double-stranded target gene, a pair of HCPs each having a region complementary to the target gene are prepared, and a Y' region and a Y region of the probes (Fig. 4, (b)) having regions complementary to a Y region and a Y' region of the double-stranded target gene shown at (a) in Fig. 4 are previously cleaved at one site each of the regions as shown at (c) in Fig. 4. The cleaved probes (Fig. 5, (d)) hybridize to the target gene in which the two strands have been separated (Fig. 5, (e)) as shown at (f) in Fig. 5, and then are ligated by means of a ligation reaction as shown at (g) in Fig. 5. Then the cleaved probes shown at (i) in Fig. 6 hybridize to the ligated probes (Fig. 6, (h)), and then are ligated by means of a ligation reaction as shown at (j) in Fig. 6.

The ligated probes (Fig. 6, (h)) and the cleaved probes (Fig. 5, (d)) are reacted so as to carry out repetitively cycles of separation, hybridization, and ligation reactions under thermal control in the range of 94 °C to 25 °C (Fig. 6, (j), Fig. 7, (k)), to form a polymer according to the principle of the PALSAR method as shown at (l) in Fig. 7.

The formed polymer can easily be detected by the general agarose gel electrophoresis or by the detecting method described hereinafter (Fig. 20 to Fig. 22).

Fig. 8 to Fig. 11 are schematic views each showing an example of detection of a double-stranded target gene by making use of ligation reactions of a pair of probes used in the PALSAR method with three sites thereof having been cleaved.

As shown at (a) and (b) in Fig. 8, from the double-stranded target gene, a pair of HCPs each having three regions complementary to the target gene are prepared, and the probes each having regions complementary to an X region or an X' region, a Y region or a Y' region, and a Z region or a Z' region in the double-stranded target gene shown at (a) in Fig. 8 (Fig. 8, (b)) are previously cleaved at one site each of the regions (at three sites in all) as shown at (c) in Fig. 8. The cleaved probes (Fig. 8, (c)) hybridize to the separated target genes as shown at (d), (e) and (f) in Fig. 9, and are ligated by means of a ligation reaction.

Then the ligated probes (Fig. 10, (g)) and the probes with the three sites each of the probes having previously been cleaved (Fig. 10, (h)) are reacted so as to carry out repetitively cycles of separation, hybridization and ligation reactions under thermal control in the range of 25 °C to 94 °C (Fig. 10, (i), (j), (k), and Fig. 11, (l)), to form a polymer according to the principle of the PALSAR method as shown at (m) in Fig. 11.

The formed polymer can easily be detected by means of the general agarose gel electrophoresis or by the detection method described hereinafter (Fig. 20 to Fig. 22).

Fig. 12 and Fig. 13 are schematic views each showing an example of detection of SNPs by making use of ligation relations of a pair of probes used in the PALSAR method with one site thereof having been cleaved.

Fig. 12 shows at (a) a pair of probes used in the PALSAR method, one probe having a region (a Y region) complementary to a target gene including SNPs with the region having been cleaved at one site.

As shown at (b1) in Fig. 12, when SNPs in the target gene including SNPs completely match (Complete match) a joined part of the probe with one site thereof having been cleaved upon hybridization, the cleaved probes are ligated to each other by means of a ligation reaction, but when SNPs in the target gene including SNPs do not completely match (Mismatch) a joined part of the probe with one site thereof having been cleaved, any ligation reaction does not occur even if hybridized, and the probes are kept in the cleaved state (Fig. 12, (b2)).

The probes not having been involved in any ligation reaction can be changed to forms incapable of responding to any ligation reaction by dephosphorylation of the probes with alkaline phosphatase as shown at (c) in Fig. 13.

The ligated probes (Fig. 13, (d)) react to another HCPs added thereto (Fig. 13, (e)), and the probes form a polymer according to the principle of the PALSAR method as shown at (f) in Fig. 13.

The formed polymer can easily be detected by the general agarose gel electrophoresis or by the detection method described hereinafter (Fig. 20 to Fig. 22).

Fig. 14 is an explanatory view showing a device advantageously used in the method according to the present invention for controlling reaction temperatures. This figure shows the I-CORE™ (Smart Cycler™) made by Cepheid Inc., and this reaction temperature control unit has a main body of vessel with approximately pentagonal side face comprising a pair of sidewalls with high thermal conductivity and inclined bottom plates inclined to and jointed to each other. Because of the construction as described above, cooling and heating may be repeatedly carried out via the sidewalls as shown at (a) in Fig. 14. Also as shown at (b) in Fig. 14, whether a fluorescent substance is present or not can be checked by projecting excited light from one of the inclined bottom plates and monitoring the state of luminescence from the other inclined bottom plate.

Fig. 15 to Fig. 19 are schematic views each showing an example of detection of a double-stranded target gene by making use of ligation reactions of a pair of probes used in the PALSAR method with one site thereof having been cleaved as described above by using the I-CORE™ (Smart Cycler™) made by Cepheid Inc.

The target gene (Fig. 15, (a)) and the pair of probes used in the PALSAR method with one site thereof having been cleaved (Fig. 15, (b)) are added in the reaction cell shown at (c) in Fig. 15.

Only when the target gene to be detected is present in the thermal-controllable reaction cell (Fig. 16, (d)), a hybridization reaction occurs between the separated target genes (Fig. 16, (e)) and the cleaved probes (as shown at (f) in Fig. 16), and the probes are ligated by means of a ligation reaction (Fig. 16, (g)). The ligated probes (Fig. 17, (h)) and the cleaved probes (Fig. 17, (i)) are reacted so as to carry out repetitively cycles of separation, hybridization and ligation reactions under thermal control in the range of 94 °C to 25 °C (as shown at (j) in Fig. 17 and at (k) in Fig. 18) to form a polymer according to the principle of PALSAR method as shown at (l) in Fig. 18.

The polymer formed as described above (Fig. 19, (m)) can be detected by adding a coloring matter that is inserted into two strands of oligonucleotide to emit fluorescence, and monitoring emission of fluorescence with the I-CORE™ (Smart Cycler™) made by Cepheid Inc. (Fig. 19, (n)).

The formed polymer can easily be detected by the general agarose gel electrophoresis or by the detection method described hereinafter (Fig. 20 to Fig. 22).

Fig. 20 to Fig. 22 are schematic views each showing an example of detection of a polymer formed by the PALSAR method with Molecular Beacons.

Each probe used in the PALSAR method is cleaved at one site each of regions (a Y region and a Y region) complementary to a target gene, as shown at (a) in Fig. 20 and at (a) in Fig. 21, with 5' ends of the HCPs being labeled with fluorescent-substances and 3' ends thereof being bound to quenchers (Fig. 20, (b), and Fig. 21, (b)). When the target gene and the complementary regions (the Y region and the Y' region) mismatch (as shown at (b) in Fig. 20), formation of any polymer according to the PALSAR method is not achieved with the fluorescence continued, but when the target gene and the complementary regions (the Y region and the Y' region) completely match each other (as shown at (b) in Fig. 21), HCPs are formed through ligation reactions (Fig. 22, (c)), so that a polymer is formed according to the principle of PALSAR method as shown at (d) in Fig. 22, and further the 5' end and the 3' end are positioned close to each other, and the fluorescence disappears because of existence of the quencher, so that the target gene can be detected. It should be noted that also a method using an enhancer in place of a quencher may be used.

In the figures, an arrow at the distal end indicates the 3' end, and a black dot at the starting end indicates the 5' end.

### Examples

Examples of the present invention are described hereinafter, and it is needles to say that the present invention is not limited to the Examples described below.

DNA probes and target genes used in Examples 1 to 4 and Comparative Example 1 to 5
a) Probe 1 (INT-1) : (5'-X'-Y'-Z'-3') 
5' - ACGCAGAAAGCGTCTAGC - CGCTCACAGTTAAG CCGTGAGATTTCAC

- GTACTGCCTGATAGGGTG - 3'
b) Probe 2 (INT-2) : (5'-X-Y-Z-3') 
5' - GCTAGACGCTTTCTGCGT - GTGAAATCTCACGG CTTAACTGTGAGCG
- CACCCTATCAGGCAGTAC - 3'
c) Probe 3 (INT-1-A) : (5'-X'-Y'(cleaved)-3') 5' - ACGCAGAAAGCGTCTAGC - CGCTCACAGTTAAG- 3'
d) Probe 4 (INT-1-B) : (5'-Y'(cleaved)-Z'-3') 5' (phosphorylated) - CCGTGAGATTTCAC - GTACTGCCTGATAGGGTG - 3'
e) Probe 5 (INT-2-C) : (5'-X-Y(cleaved)-3') 5' - GCTAGACGCTTTCTGCGT - GTGAAATCTCACGG- 3'
f) Probe 6 (INT-2-D) : (5'-Y'(cleaved)-Z-3') 5' (phosphorylated) - CTTAACTGTGAGCG - CACCCTATCAGGCAGTAC - 3'
g) Target gene (INT-①) : (5'--Y--3') 
5' - GGTTTGTCGCGTTGTTC - GTGAAATCTCACG G C TTAACTGTGAGCG

- TGCGGGCGATACGGG - 3 '
h) Target gene (INT-①S1) : (5'--Y--3') 
5' - GGTTTGTCGCGTTGTTC - GTGAAATCTCACG T C TTAACTGTGAGCG

- TGCGGGCGATACGGG - 3 '
i) Target gene (INT-①S2) : (5'--Y--3') 
5' - GGTTTGTCGCGTTGTTC - GTGAAATCTCACG G G TTAACTGTGAGCG

- TGCGGGCGATACGGG - 3 '
j) Target gene (INT-①R): (5'--Y--3')

5 '- CCCGTATCGCCCGCA - CGCTCACAGTTAAG CCGTGAGATTTCAC
- GAACAACGCGACAAACC - 3'

### (Example 1, Comparative Example 1, and Comparative Example 2) Ligation reactions in HCPs (Target gene: double-stranded target gene)

### 1: Object

It was tried to detect a double-stranded target gene by means of ligation reactions of HCPs, each probe having been cleaved at one site.

### 2: Materials

1) 60-bases of synthetic oligonucleotide derived from Mycobacterium intracellular 16s rRNA gene (described as INT-① hereinafter) and synthetic oligonucleotide complementary to the target gene INT-① (described as INT-① R hereinafter) as the target gene were prepared to form two strands, and the following experiment was carried out using the double-stranded target gene.
2) A pair of HCPs comprising a probe 1 having a region complementary to a Y region of the target gene INT-① (described as INT-1 hereinafter) and a probe 2 in which each region is complementary to that of the probe 1 (described as INT-2 hereinafter) were prepared. Further probes obtained by cleaving one site each of the probes were prepared (described as INT-1-A, INT-1-B, INT-2-C, and INT-2-D).
3) Tsc DNA ligase, thermostable (made by Roche Molecular Biochemicals) was used as thermostable DNA ligase, and a buffer attached thereto was used as an incubation buffer.
4) 20 x SSC (3M-NaCl, 0.3 M - C₆H₅O₇Na₃, 2H₂O, pH 7.0) was used as a buffer.

### 3. Method

### a) Preparation of reaction solutions

The INT-1-A, INT-1-B, INT-2-C and INT-2-D each prepared to the concentration of 100 pmol/µL were added by 0.5 µL to 0.2 mL sterilized micro tubes, respectively; 1 µL of ligase and 2 µL of incubation buffer were added; and further 1 µL of the double-stranded target gene comprising INT-① and INT-①R each prepared to the concentration of 100 pmol/µL was added to the mixtures; and the mixtures were diluted with DW² (sterilized redistilled water) to obtain 20µL reaction solutions, respectively (Example 1). Further for control, a reaction solution with no target gene added therein (Comparative Example 1) and a reaction solution with neither ligase nor target gene added therein (Comparative Example 2) were prepared.

### b) Ligation reactions

Then the reaction solutions were reacted at 94 °C for two minutes with a thermal cycler (produced by Perkins Elmer Inc.), respectively, followed by 30 cycles of heating [for 30 seconds at 94 °C, → for 2 minutes at 50 °C, → and 5 minutes at 55 °C ] in all for achieving ligation reactions. Then a reaction for inactivating the ligase was performed for 10 minutes at 99 °C.

### c) Detection of ligation by means of polyacrylamide gel electrophoresis (PAGE)

After the ligation reactions, PAGE was performed for each reaction solution. 5µL of each reaction solution were subjected to electrophoresis with 16% PAGE (30% Acrylamide/Bis solution 29:1, 10% Ammonium Persulfate, TEMED; Bio-Rad laboratories, 10% TBE, H₂O) for 90 minutes at 130 V. As controls, INT-①, INT-①R and the double-stranded target gene comprising INT-① and INT-①R were used. A 10 bp (base pairs) of DNA ladder (produced by GIBCO BRL Inc.) was used as a molecule size marker. After electrophoresis was continued for 90 minutes, the gel was stained with ethidium bromide (EtBr solution produced by Nippon Gene Inc.).

### d) Reactions for forming a polymer

10µL of 20 x SSC were added to 10µL of reaction solutions having been subjected to the ligation reactions in 0.2 mL microtubes and the reaction was continued for 16 hours at 66 °C with the thermal cycler (produced by Perkins Elmer Inc.).

### e) Detection of the polymer by means of the agarose gel electrophoresis

8µL of the reaction solutions having completed the reaction for forming a polymer were subjected to electrophoresis for 30 minutes at 100 V with 2% Nusieve 3 : 1 agarose gel (produced by Bio Whittaker Molecular Applications Inc.). A 1 Kb of DNA Extension Ladder (produced by GIBCO BRL Inc.) was used as a molecular size marker. After completion of the agarose gel electrophoresis, the gel was stained with ethidium bromide.

### 4. Result

The result is shown at (a) and (b) in Fig. 23. From a result of the PAGE performed to detect a result of ligation shown at (a) in Fig. 23, a band not observed in the control (Comparative Example 2, lane 2) was detected at a high position indicated by the arrow in the figure when the target gene was added (Example 1, lane 1). This band seems to be that for the ligated probes produced by the ligation reactions. The same result as that in Comparative Example 2 was obtained also in Comparative Example 1, although not shown in the figure.

As shown at (b) in Fig. 23, in the experiment for detecting formation of the polymer by means of the agarose gel electrophoresis, formation of the polymer was observed only when the target gene was added (Example 1, lane 1), and any polymer was not observed in Comparative Example 1 (lane 2), not in Comparative Example 2 (lane 3). As described above, it has been confirmed that cleaved HCPs are ligated by the double-stranded target gene, and that the double-stranded target gene can be detected by checking a result of polymer formation by the ligated HCPs.

### (Examples 2 to 4, and Comparative Example 3) Detection of SNPs by means of ligation reactions of HCPs

### 1. Object

It was investigated whether a single-stranded target gene and SNPs can be detected by means of ligation reactions of HCPs, each probe having been cleaved at one site or not.

### 2. Materials

In addition to the probes, ligase, ligation buffer, buffer solution, and target gene INT-① used in Example 1, target genes with only one base in the Y region in INT-① having been changed to another one; namely INT-①S1 ( in which one base of the part complementary to the 5' end of the Y region in the cleaved HCP is different) and INT-①S2 (in which one base of the part complementary to the 3' end of the Y region in the cleaved HCP) were newly prepared.

### 3. Method

### a) Preparation of reaction solutions

INT-1-A, INT-1-B, INT-2-C and INT-2-D each prepared to the concentration of 100 pmol/µL were added by 0.5µL to sterilized 0.2 mL microtubes, respectively; 1µL of ligase and 2 µL of incubation buffer were added to the samples; and further the target gene prepared to the concentration of 100pmol/µL was added by 0.5 µL thereto; and the resultant mixtures were diluted by DW² to obtain 20 µL reaction solutions, respectively. As a target gene, the INT-① was selected in Example 2 and INT-①S1 in Example 3, and in INT-①S2 in Example 4, respectively. For control, also reaction solutions (Comparative Example 3) were prepared by not adding any target genes (INT-①, INT-①S1, and INT-①S2) to the above reaction solutions.

### b) Ligation reactions

Then the reaction solutions were reacted so as to carry out 30 cycles of heating [for 30 seconds at 94 °C, →for 90 seconds at 50 °C, →and 3 minutes at 55 °C] with the thermal cycler (produced by Perkins Elmer Inc.) to achieve ligation reactions. Then a reaction for inactivating the ligase was performed for 10 minutes at 99 °C.

### c) Detection by polyacrylamide gel electrophoresis (PAGE)

PAGE was carried out for detection in each reaction solution by the same procedure and under the same conditions as those in Example 1. As controls, INT-①, INT-①S1, and INT-①S2 were used. A 50 bp of DNA ladder (produced by GIBCO BRL Inc.) was used, in addition to the 10 bp of DNA ladder, as a molecular size marker.

### d) Reactions for forming a polymer

Each of the reaction solutions after the ligation reactions was subjected to a reaction for forming a polymer by the same procedure and under the same conditions as those in Example 1.

### e) Detection of the polymer by means of the agarose gel electrophoresis

Each of the reaction solutions having been subjected to the reaction for forming the polymer was then subjected to the agarose gel electrophoresis by the same procedure and under the same conditions as those in Example 1.

### 4. Result

The result is shown at (a) and (b) in Fig. 24. As shown in the result of the PAGE performed for detection of ligation shown at (a) in Fig. 24, in the reaction solution with INT-① added therein (Example 2, lane 1), sequences between the target gene and the Y region of the cleaved probe completely match each other, so that ligation reactions proceeded, and the dense band indicated by the arrow was observed at a position higher than about 350 bp. On the contrary, in the reaction solution with INT-①S1 (Example 3, lane 2) and INT-①S2 (Example 4, lane 3) containing SNPs added therein, respectively, as sequences between the target gene and the Y region of the cleaved probe were different from each other in one base (mismatch), any ligation did not occur, and the band observed in lane 1 was not detected. On the other hand, in lane 3 where only the ligase was added and any target gene was not added (Comparative Example 3), only the dense band formed by the four cleaved probes linked to each other in a circular form was observed.

As shown at (b) in Fig. 24, also in the experiment for detecting formation of the polymer by the agarose gel electrophoresis, the polymer was formed only in the reaction solution with the INT-①, in which sequences between the target gene and the Y region of the cleaved probes completely matched each other, added therein (Example 2, lane 1), and any polymer was not observed in Example 3 (lane 2), nor in Example 4 (lane 3). Although not shown herein, any polymer was not observed also in Comparative Example 3 as in Examples 3 and 4. As described above, it has been confirmed that the cleaved HCPs are ligated by a single-stranded target gene having the completely matching sequence, and that the single-stranded target gene can be detected by checking formation of the polymer by the ligated HCPs. Further, in SNPs, the cleaved HCPs are not ligated, and any polymer is not formed, and therefore it was confirmed that SNPs can be detected by checking ligation of HCPs with one site thereof having been cleaved.

### (Comparative Example 4 and Comparative Example 5)

### 1. Object

A result of the reaction for forming a polymer by means of the PALSAR method in the case where the HCPs were cleaved was compared to that in the case where the HCPs were not cleaved.

### 2. Materials

1) INT-1 and INT-2 were used as probes in Comparative Example 4, while INT-1-A, INT-1-B, INT-2-C, and INT-2-D were used as probes in Comparative Example 5.
2) As a buffer solution, 20 x SSC (3M NaCl 0.3M C₆H₅O₇Na₃ · 2H₂O, pH 7.0) was used.

### 3. Method

### a) Preparation of reaction solutions

0.5µL of probes each prepared to the concentration of 100 pmol/µL and 20 µL of 20 x SSC were added to 0.2 mL sterilized microtubes, respectively, and 20 µL of reaction solutions were obtained with DW². INT-1 and INT-2 were used as probes in Comparative Example 4, and INT-1-A, INT-1-B, INT-2-C, and INT-2-D were used as probes in Comparative Example 5.

### b) Reactions for forming a polymer

Each of the reaction solutions were reacted for 30 seconds at 94 °C with the thermal cycler (produced by Perkins Elmer Inc.), and the reaction was continued for 16 hours at 66 °C.

### c) Detection of the polymer by means of the agarose gel electrophoresis

Agarose gel electrophoresis was performed to each reaction solution by the same procedure and under the same conditions as those in Example 1.

### 4. Result

As shown at (b) in Fig. 23 and at (b) in Fig. 24, formation of the polymer was observed in Comparative Example 4 in which probes not having been cleaved were used, while formation of any polymer was not observed in Comparative Example 5 in which cleaved probes were used. (Experimental Example 1) Reactions for forming a polymer using HCPs by the PALSAR method

### 1. Object

A result of a reaction for forming a polymer in the case where a pair of probes used in the PALSAR method are cleaved at one site each of the probes, is compared, changing a temperature in the reactions, to that in the case where a pair of probes used in the PALSAR method are not cleaved.

### 2. Materials

1) The following pair of HCPs (probe 7, probe 8) and probes 9, 10 each obtained by cleaving the probe 7 at one site were used for polymerization.
   a) Probe 7 (HCP-1) 
5' - GAATAAGTCATAGCTCATA - AAGGACTGG TCGCTCGAC
- ATCGTCCTAGGGAGATTCG - 3'
   b) Probe 8 (HCP-2) 
5' - TATGAGCTATGACTTATTC - GTCGAGCGACCAGTCCTT
- CGAATCTCCCTAGGACGAT - 3'
   c) Probe 9 (HCP-1-1) 5' - GAATAAGTCATAGCTCATA - AAGGACTGG - 3'
   d) Probe 10 (HCP-1-2) 5' (phosphorylated) - TCGCTCGAC - ATCGTCCTAGGGAGATTCG - 3'
2) 20 x SSC (3M NaCl, 0.3M C₆H₅O₇Na₃ · 2H₂O, pH 7.0) was used as a buffer solution.

### 3. Method

Probe 7 and probe 8, or probe 9, probe 10 and probe8 each prepared to the concentration of 10¹³ copies/µL were added by 0.5µL to 0.2 mL sterilized microtubes, respectively; 40µL of 20 x SSC were additionally added to each reaction solution with a cover set on each microtube, and then the reaction solutions were boiled for 30 seconds at 94 °C and additionally heated for 30 minutes at 52 °C, 54°C, 56 °C, 58 °C, 60 °C, 62 °C, 64 °C, 66 °C, 68 °C, and 70 °C, respectively.

After heating, electrophoresis was performed for each reaction solution by using 0.5% agarose gel, and results of polymerization in each reaction solution were checked by staining with ethidium bromide.

### 4. Result

Fig. 27 and Fig. 28 are photographs each showing a result of Experimental Example 1 in which electrophoresis was performed for 30 minutes with a 0.5 % agarose gel at 100 V.

Fig. 27 is a photograph showing a result in the case where the pair of probes used in the PALSAR method was not cleaved. As indicated by the arrow in Fig. 27, the double-stranded polymer was formed depending on the temperature for hybridization in the case of HCPs not having been cleaved.

Fig. 28 is a photograph showing a result in the case where the pair of probes used in the PALSAR method was cleaved at one site. As shown in Fig. 28, any polymer was not formed at any temperature for hybridization.

### Capability of Exploitation in Industry:

As described above, by making use of the gene detecting method according to the present invention, a gene can be detected by checking formation of the polymer without the need of catching linked oligonucleotides nor of washing to remove surplus oligonucleotides by making use of the PALSAR method, and further by labeling the 5' ends of the pair of probes used in the PALSAR method with fluorescent substances and binding the 3' ends thereof to quenchers, it is possible to advantageously detect, without the need of any specific enzyme, a gene by checking disappearance of fluorescence by the quenchers when the double-stranded polymer is formed by means of the PALSAR method.

## Claims

1. A gene detecting method by making use of a probe-polymer forming method for forming a double-stranded polymer by providing a plurality of pairs of probes, each probe comprising n (n ≧ 3) base sequence regions complementary to each other, and hybridizing the probes so as to cross alternately, wherein the gene detecting method comprises the steps of:
providing the plurality of pairs of probes, wherein one probe of the pair of probes comprises at least one complementary region having base sequence complementary to a part of a target gene;
previously cleaving the region or the regions and/or a partner or partners thereof at least at one site, the region or the regions being complementary to the target gene of the pair of probes;
performing hybridization reactions, ligation reactions and separation reactions under thermal control;
ligating the cleaved probes to form complete probes; and
forming the double-stranded polymer by means of the probe-polymer forming method to detect the target gene.

2. A method according to claim 1, wherein the ligation reactions are performed by means of DNA ligase, thermostable DNA ligase or autoligation not using any enzyme.

3. A method according to claim 1 or 2, wherein a single-stranded DNA or RNA is used as the target gene.

4. A method according to claim 1 or 2, wherein a double-stranded DNA or RNA is used as the target gene.

5. A method according to any of claims 1 to 4, wherein the previously cleaved sites of the probes are designed to be complementary to SNPs.

6. A method according to any of claims 1 to 5, wherein a device for controlling reaction temperature is used.

7. A method according to any of claims 1 to 6, wherein at least one quencher and at least one fluorescent substance are bounded to one or both ends of the pair of probes to be used and formation of the double-stranded polymer is detected in response to fluorescence quenching.


SEQUENCE LISTING

<110> Sanko Junyaku Co., Ltd.

<120> Gene detecting method

<130> 76014-PCT-EP

<140> PCT/JP01/07020
<141> 2001-08-14

<150> JP 2000-261687
<151> 2000-08-30

<160> 14

<210> 1
<211> 64
<212> DNA
<213> Artificial Sequence

<220>
<223> Description of Artificial Sequence: probe 1

<400> 1
acgcagaaag cgtctagccg ctcacagtta agccgtgaga tttcacgtac tgcctgatag 60

ggtg 64

<210> 2
<211> 64
<212> DNA
<213> Artificial Sequence

<220>
<223> Description of Artificial Sequence: probe 2

<400> 2
gctagacgct ttctgcgtgt gaaatctcac ggcttaactg tgagcgcacc ctatcaggca 60

gtac 64

<210> 3
<211> 32
<212> DNA
<213> Artificial Sequence

<220>
<223> Description of Artificial Sequence: probe 3

<400> 3
acgcagaaag cgtctagccg ctcacagtta ag 32

<210> 4
<211> 32
<212> DNA
<213> Artificial Sequence

<220>
<221> misc-feature
<222> (1)
<223> phosphoric acid attached at the 5' end

<220>
<223> Description of Artificial Sequence: probe 4

<400> 4
ccgtgagatt tcacgtactg cctgataggg tg                                32

<210> 5
<211> 32

<212> DNA
<213> Artificial Sequence

<220>
<223> Description of Artificial Sequence: probe 5

<400> 5
gctagacgct ttctgcgtgt gaaatctcac gg                                32

<210> 6
<211> 32
<212> DNA
<213> Artificial Sequence

<220>
<221> misc-feature
<222> (1)
<223> phosphoric acid attached at the 5' end

<220>
<223> Description of Artificial Sequence: probe 6

<400> 6
cttaactgtg agcgcaccct atcaggcagt ac                                32

<210> 7
<211> 60
<212> DNA
<213> Artificial Sequence

<220>
<223> Description of Artificial Sequence: target gene-1

<400> 7

ggtttgtcgc gttgttcgtg aaatctcacg gcttaactgt gagcgtgcgg gcgatacggg  60

<210> 8
<211> 60
<212> DNA
<213> Artificial Sequence

<220>
<223> Description of Artificial Sequence: target gene-S1

<400> 8
ggtttgtcgc gttgttcgtg aaatctcacg tcttaactgt gagcgtgcgg gcgatacggg  60

<210> 9
<211> 60
<212> DNA
<213> Artificial Sequence

<220>

<223> Description of Artificial Sequence: target gene-S2

<400> 9
ggtttgtcgc gttgttcgtg aaatctcacg ggttaactgt gagcgtgcgg gcgatacggg  60

<210> 10
<211> 60
<212> DNA
<213> Artificial Sequence

<220>
<223> Description of Artificial Sequence: target gene-1R

<400> 10
cccgtatcgc ccgcacgctc acagttaag ccgtgagattt cacgaacaac gcgacaaacc  60

<210> 11
<211> 56
<212> DNA
<213> Artificial Sequence

<220>
<223> Description of Artificial Sequence: probe 7

<400> 11
gaataagtca tagctcataa aggactggtc gctcgacatc gtcctaggga gattcg      56

<210> 12
<211> 56
<212> DNA
<213> Artificial Sequence

<220>
<223> Description of Artificial Sequence: probe 8

<400> 12
tatgagctat gacttattcg tcgagcgacc agtccttcga atctccctag gacgat      56

<210> 13
<211> 28
<212> DNA
<213> Artificial Sequence

<220>
<223> Description of Artificial Sequence: probe 9

<400> 13
gaataagtca tagctcataa aggactgg                                     28

<210> 14

<211> 28
<212> DNA
<213> Artificial Sequence

<220>
<221> misc-feature
<222> (1)
<223> phosphoric acid attached at the 5' end

<220>
<223> Description of Artificial Sequence: probe 10

<400> 14
tcgctcgaca tcgtcctagg gagattcg 28